(19) Europäisches Patentamt
European Patent Office
Office européen des brevets

(11) Veröffentlichungsnummer: **0 257 251**
**B1**

# (12) EUROPÄISCHE PATENTSCHRIFT

(45) Veröffentlichungstag der Patentschrift: **11.07.90**

(51) Int. Cl.⁵: **C07C 205/04**, C07C 201/12, A01N 33/20

(21) Anmeldenummer: **87109690.5**

(22) Anmeldetag: **06.07.87**

## (54) Fluoralkyl enthaltende Dimethylolnitromethane, Verfahren zu ihrer Herstellung sowie ihre Verwendung.

(30) Priorität: **09.07.86 DE 3622976**

(43) Veröffentlichungstag der Anmeldung:
**02.03.88 Patentblatt 88/9**

(45) Bekanntmachung des Hinweises auf die Patenterteilung:
**11.07.90 Patentblatt 90/28**

(84) Benannte Vertragsstaaten:
**BE CH DE FR GB IT LI NL SE**

(56) Entgegenhaltungen:
**EP-A- 0 116 885**
**DE-A- 1 804 068**
**DE-A- 1 954 173**
**DE-A- 2 853 040**

**CHEMICAL ABSTRACTS, Band 62, Nr. 1, 4. Januar 1965, Spalte 430c, Columbus, Ohio, US; I.L. KNUNYANTS "Aliphatic fluoro nitro compounds. III. Fluorine-containing nitro alcohols and ethers"**
**CHEMICAL ABSTRACTS, Band 64, Nr. 1, 3. Januar 1966, Spalte 731c, Columbus, Ohio, US; L.W. KISSINGER "Action of polyphosphoric acid on 2-nitro-1,3-propanediols and some of their carbonate, sulfite, and 1,3-dioxane derivatives"**

(73) Patentinhaber: **BAYER AG,**
**D-5090 Leverkusen 1 Bayerwerk(DE)**

(72) Erfinder: **Baasner, Bernd, Dr., Hamberger Strasse 27d, D-5090 Leverkusen 3(DE)**
Erfinder: **Heywang, Gerhard, Dr., Nittumer Weg 4, D-5060 Bergisch-Gladbach 2(DE)**
Erfinder: **Kühle, Engelbert, Dr., Von Bodelschwingh Strasse 42, D-5060 Bergisch-Gladbach 2(DE)**
Erfinder: **Paulus, Wilfried, Dr., Deswatinesstrasse 90, D-4150 Krefeld 1(DE)**
Erfinder: **Schmitt, Hans-Georg, Dr., Am Oberend 13, D-4150 Krefeld(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue, fluoralkylhaltige Dimethylolnitromethane, ein Verfahren zu ihrer Herstellung sowie die Verwendung von fluoralkylhaltigen Dimethylolnitromethanen im Materialschutz.

2-Nitro-2-trifluormethylpropan-1,3-diol sowie dessen Herstellung ist aus CA 62, 430c (1965) bekannt. Weiterhin ist bekannt, daß bestimmte Methylolnitromethan-Derivate mikrobizide Eigenschaften aufweisen (vgl. z.B. W. Paulus in Biodet. Proceed. of the IV. Internat. Symp., Berlin; Pitman Publishing Ltd., London 1980, S. 312 sowie DE-OS 1 804 068 und DE-OS 1 954 173). Die mirkobizide Wirkung dieser Verbindungen ist jedoch in bestimmten Indikationsgebieten, insbesondere bei niedrigen Aufwandmengen und -konzentrationen, nicht immer befriedigend.

Es wurden nun neue Fluoralkyl enthaltende Dimethylolnitromethane der Formel (I)

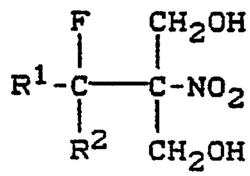

(I),

worin $R^1$ und $R^2$ gleich oder verschieden sind und Wasserstoff, Halogen, Alkyl oder Halogenalkyl bedeuten, wobei die Verbindung ausgenommen ist, bei der $R^1$ und $R^2$ für Fluor stehen,
gefunden.

Als Halogene für die Verbindungen der Formel (I) seien genannt: Fluor, Chlor, Brom und Iod, bevorzugt Fluor, Chlor und Brom.

Als Alkylgruppen seien solche mit 1 bis 6, bevorzugt mit 1 bis 4, Kohlenstoffatomen genannt, wie Methyl, Ethyl, Propyl und Butyl sowie deren verzweigte Isomere.

Als Halogenalkylgruppen seien solche mit 1 bis 4, bevorzugt 1 bis 2, Kohlenstoffatomen genannt, die ein- oder mehrfach durch Fluor, Chlor, Brom oder Iod, bevorzugt durch Fluor oder Chlor, substituiert sind. Genannt seien beispielsweise Chlormethyl, Dichlormethyl, Trichlormethyl, Fluormethyl und Difluormethyl.

Bevorzugt sind solche Dimethylolnitromethane der Formel (I), in denen $R^1$ und $R^2$ gleich oder verschieden sind und für Wasserstoff, Fluor, Chlor, Brom, Methyl, Ethyl, Propyl, Fluormethyl, Difluormethyl, Trifluormethyl, Chlormethyl, Dichlormethyl sowie Trichlormethyl stehen.

Zum Beispiel seien folgende Verbindungen genannt:

2-Nitro-2-(chlorfluormethyl)propan-1,3-diol,
2-Nitro-2-(dichlorfluormethyl)propan-1,3-diol,
2-Nitro-2-(difluormethyl)propan-1,3-diol,
2-Nitro-2-(bromfluormethyl)propan-1,3-diol,
2-Nitro-2-(fluormethyl)propan-1,3-diol,
2-Nitro-2-(chlordifluor)propan-1,3-diol,
3-Fluor-2-hydroxymethyl-2-nitro-butanol-1,
3,3-Difluor-2-hydroxymethyl-2-nitro-butanol-1,
3,4-Difluor-3-fluormethyl-2-hydroxymethyl-2-nitro-butanol-1,
4-Chlor-3-fluor-3-chlormethyl-2-hydroxymethyl-2-nitro-butanol-1 und
4-Chlor-3-fluor-3-fluormethyl-2-hydroxymethyl-2-nitro-butanol-1.

Gegenstand der vorliegenden Erfindung ist auch ein Verfahren zur Herstellung von Fluoralkyl enthaltenden Dimethylolnitromethanen der Formel (I)

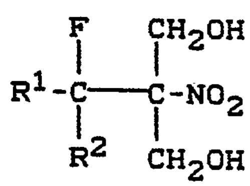

(I),

worin $R^1$ und $R^2$ gleich oder verschieden sind und Wasserstoff, Halogen, Alkyl oder Halogenalky bedeuten, wobei die Verbindung ausgenommen ist, bei der $R^1$ und $R^2$ für Fluor stehen,
das dadurch gekennzeichnet ist, daß man Nitroalkane der Formel (II)

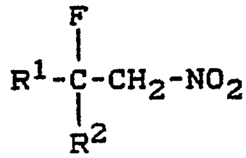

(II),

worin $R^1$ und $R^2$ die oben angegebene Bedeutung besitzen, mit Formaldehyd der Formel (III)

$CH_2O$(III),

oder mit cyclischen Formaldehydderivaten der Formel (IV),

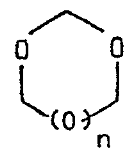

(IV),

worin n für 1 oder 2 steht,
oder mit Polyformaldehyden der Formel (V),

$(CH_2\text{-}O\text{-})_n$(V),

worin n für eine Zahl >10, bevorzugt 100 bis 10.000, steht,
in Gegenwart eines Lösungs- und/oder Verdünnungsmittels und gegebenenfalls in Gegenwart einer Base umsetzt.

Setzt man beispielsweise 2-Chlor-2,2-difluornitroethan mit Formaldehyd um, so kann der Reaktionsablauf des erfindungsgemäßen Verfahrens durch das folgende Formelschema wiedergegeben werden:

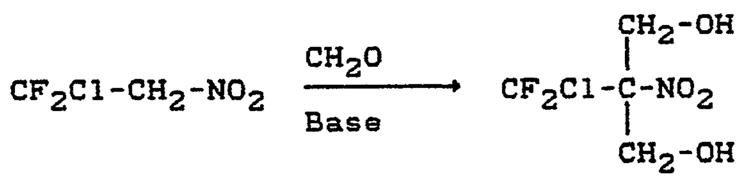


Die bei der Durchführung des erfindungsgemäßen Verfahrens als Ausgangsstoffe eingesetzten fluorhaltigen Nitroalkane der Formel (II) sind bekannt. Sie können beispielsweise nach den in den deutschen Offenlegungsschriften 3 305 201 und 3 305 202 angegebenen Verfahren hergestellt werden.

Die Wahl der Art des Lösungs- und/oder Verdünnungsmittels unterliegt keinen Einschränkungen. Beispielsweise können als Lösungs- und/oder Verdünnungsmittel eingesetzt werden: Wasser, Alkohole, wie Methanol oder Ethanol, Nitrile, wie Acetonitril, Ether, wie Tetrahydrofuran, aromatische Verbindungen, wie Toluol, bevorzugt sind Wasser, Methanol und Ethanol. Die Menge an einzusetzenden Lösungs- und/oder Verdünnungsmitteln ist nicht kritisch und kann leicht durch geeignete Vorversuche ermittelt werden.

Als Basen können alle üblichen organischen und anorganischen Basen eingesetzt werden. Hierzu gehören vorzugsweise tertiäre Amine, Alkalihydroxide, Alkalicarbonate, Alkalihydrogencarbonate sowie Alkali-fluoride. Beispielsweise seien bevorzugt genannt: Triethylamin, Natriumhydroxid, Kaliumhydroxid, Natriumhydorgencarbonat, Kaliumcarbonat und Kaliumfluorid.

Die Reaktionstemperaturen können bei der Durchführung des erfindungsgemäßen Verfahrens in einem größeren Bereich variiert werden. Im allgemeinen arbeitet man bei Temperaturen von etwa -70°C bis 150°C, vorzugsweise von -30°C bis 100°C.

Die Nitroalkane der Formel (II) und der Formaldehyd oder dessen Derivate der Formeln (IV) und (V) werden nach dem erfindungsgemäßen Verfahren vorzugsweise im Verhältnis von etwa 1:2-8 eingesetzt, ganz besonders bevorzugt 1:2-4.

Die Isolierung der neuen Dimethylolnitromethane der allgemeinen Formel (I) erfolgt in allgemein üblicher Art und Weise beispielsweise durch Versetzen des Reaktionsgemisches mit Wasser, Extraktion mit einem organischen Lösungsmittel, Chromatographie oder Destillation. Teil weise fallen die Produkte direkt kristallin an und können durch Filtration gereinigt werden.

Es wurde weiterhin gefunden, daß die fluoralkylhaltigen Dimethylolnitromethane der Formel (VI)

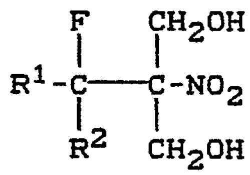 (VI),

worin $R^1$ und $R^2$ gleich oder verschieden sind und für Wasserstoff, Halogen, Alkyl oder Halogenalkyl stehen,
sehr gut antimikrobiell wirksam sind und zudem über ein breites und ausgeglichenes Wirkungsspektrum verfügen, das Bakterien, Hefen, Schimmelpilze und Schleimorganismen umfaßt. Die erfindungsgemäßen Substanzen der Formel (VI) können daher mit Vorteil in mikrobiziden Mitteln zum Schutz technischer Materialien verwendet werden. Sie sind effektiver und breiter wirksam als beispielsweise der bekannte Konservierungsstoff Trimethylol-nitromethan.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlschmierstoffkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Bakterien, Hefen und Pilze sowie gegen Schleimorganismen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsebiet können die erfindungsgemäßen Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen des Wirkstoffes mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allge meinen liegen die Anwendungskonzentrationen im Bereich von 0,01 bis 1 Gew.-%, vorzugsweise von 0,05 bis 0,5 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Rhodanidomethylthiobenzthiazol, Organo-Zinnverbindungen, Methylenbisthiocyanat, Phenolderivate, wie 2-Phenylphenol, (2,2'-Dihydroxy-5,5'-dichlor)-diphenylmethan und 3-Methyl-4-chlor-phenol.

<u>Herstellungsbeispiele</u>

1) 2-(Chlordifluormethyl)-2-nitro-propan-1,3-diol

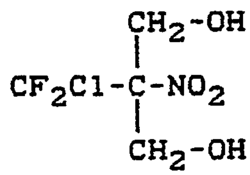

250 g Difluorchlornitroethan (1,72 Mol) wurden mit 460 ml 30 %iger Formalinlösung vereinigt und auf -10°C gekühlt, während 2 Stunden wurden bei dieser Temperatur 60 ml 10 % Natronlauge zugetropft. Anschließend ließ man das Gemisch auf Raumtemperatur erwärmen und säuerte mit 50 ml 1 N Salzsäure an. Der gebildete Niederschlag wurde abgesaugt, die Mutterlauge dreimal mit Ether extrahiert, die organische Phase über Magnesiumsulfat getrocknet und eingeengt. Der Nutschrückstand und der Feststoff aus der Etherphase wurden vereinigt und aus 100 ml Ethanol umkristallisiert. Man erhielt 201 g (57 % d.Th.) Produkt mit einem Schmelzpunkt von 85-6°C.
Auf analoge Weise wurden hergestellt:

2) 2-(Bromfluormethyl)-2-nitro-propan-1,3-diol

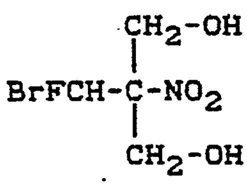

$n_D^{20}$ : 1.4924

Ausbeute 38 %

3) 2-(Difluormethyl)-2-nitro-propan-1,3-diol

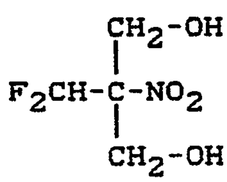

$n_D^{20}$ : 1.4822

Ausbeute 45 %

4) 2-(Dichlorfluormethyl)-2-nitro-propan-1,3-diol

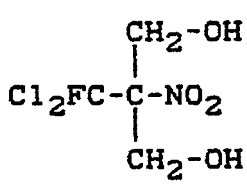

$n_D^{20}$ : 1.4792

Ausbeute 30,5 %

5) 2-(Trifluormethyl)-2-nitro-propan-1,3-diol

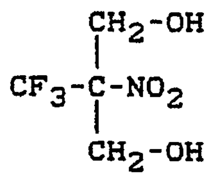

Fp. 140° C

Ausbeute 91 %

6) 2-(Chlorfluormethyl)-2-nitro-propan-1,3-diol

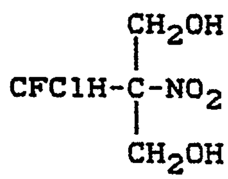

$n_D^{20}$ : 1.4870

Ausbeute 37 %

Anwendungsbeispiele

Anwendungsbeispiel 1

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % rel. Luftfeuchtigkeit wird der MHK bestimmt. MHK ist die niedrigste Konzentration an Wirkstoff, bei der keinerlei Bewuchs durch die verwendete Mikrobenart erfolgt, sie ist in der nachstehenden Tabelle angegeben.

Tabelle 1 MHK's in mg/1 bei der Einwirkung erfindungsgemäßer Substanzen auf Pilze

| Testorganismen | Substanzen erfindungsgemäß* | Vergleich** | erfindungsgemäß*** |
|---|---|---|---|
| Alternaria tenius | 200 | | 200 |
| Aspergillus niger | 500 | >1000 | 500 |
| Aureobasidium pullulans | 200 | | 350 |
| Chaetomium globosum | 500 | >1000 | 500 |
| Cladosporium cladosporioides | 200 | | 350 |
| Lentinus tigrinus | 150 | | 200 |
| Penicillium glaucum | 1000 | >4000 | 500 |
| Sclerophoma pityophila | 100 | | 200 |
| Trichoderma viride | 500 | | >1000 |

** = Trimethylol-nitro-methan

* = Dimethylol-trifluormethyl-nitro-methan

*** = 2-(Bromfluormethyl)2-nitro-propan-1,3-diol

Anwendungsbeispiel 2

Wirkung gegen Bakterien

Ein Agar, der als Nährmedium Bouillon enthält, wird mit erfindungsgemäßen Wirkstoffen in Konzentrationen von 1 bis 5000 ppm versetzt. Darauf infiziert man das Nährmedium jeweils mit den in Tabelle II aufgeführten Testorganismen und hält das infizierte Medium 2 Wochen bei 28°C und 60 bis 70 % rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle II wiedergegeben.

Tabelle II

Angabe der MHK-Werte in mg/l bei der Einwirkung der unten angegebenen Wirkstoffe auf Bakterien

| Testorganismen | MHK in mg/l der Wirkstoffe | | | |
|---|---|---|---|---|
| | erfindungsgemäß* | Vergleich** | erfindungsgemäß*** | erfindungsgemäß**** |
| Escherichia coli | 200 | 3000 | 500 | 500 |
| Staphylococcus aureus | 200 | 500 | 500 | 500 |
| Pseudomonas aeruginosa | 350 | 1000 | 500 | 500 |
| Pseudomonas fluorescens | 500 | | 200 | 500 |
| Aerobacter aerogenes | 500 | | 1000 | 500 |
| Aeromonas punctata | 500 | | 1000 | 500 |
| Bacillus mycoides | 500 | | 500 | 200 |
| Bacillus subtilis | 500 | | 500 | 200 |
| Leuconostoc mesenteroides | 500 | | 500 | 500 |
| Proteus mirabilis | 200 | | 500 | 500 |

** Trimethylol-nitro-methan
* Dimethylol-trifluormethyl-nitro-methan
*** 2-(Bromfluormethyl)-2-nitro-propan-1,3-diol
****2-(Difluormethyl)-2-nitro-propan-1,3-diol

## Patentansprüche

1. Fluoralkyl enthaltende Dimethylolnitromethane der Formel

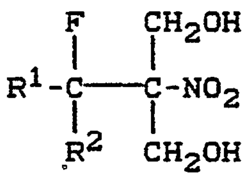
,

worin $R^1$ und $R^2$ gleich oder verschieden sind und Wasserstoff, Halogen, Alkyl oder Halogenalkyl bedeuten, wobei die Verbindung ausgenommen ist, bei der $R^1$ und $R^2$ für Fluor stehen.

2. Verfahren zur Herstellung con Fluoralkyl enthaltenden Dimethylolnitromethanen der Formel

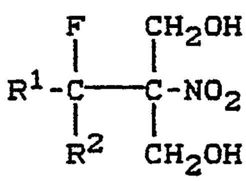
,

worin $R^1$ und $R^2$ gleich oder verschieden sind und Wasserstoff, Halogen, Alkyl oder Halogenalkyl bedeuten, wobei die Verbindung ausgenommen ist, bei der $R^1$ und $R^2$ für Fluor stehen, dadurch gekennzeichnet, daß man Nitroalkane der Formel

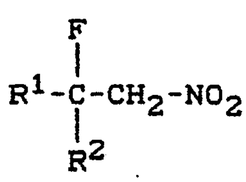
,

worin $R^1$ und $R^2$ die oben angegebene Bedeutung besitzen,
mit Formaldehyd der Formel $CH_2O$
oder mit cyclischen Formaldehydderivaten der Formel

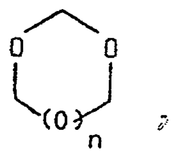

worin n für 1 oder 2 steht,
oder mit Polyformaldehyden der Formel
$(CH_2\text{-}O\text{-})_n$,
worin n für eine Zahl > 10 steht,
in Gegenwart eines Lösungs- und/oder Verdünnungsmittels und gegebenenfalls in Gegenwart einer Base umsetzt.

3. Verfahren nach Anspruch 2, dadurch gekennzeichnet, daß man die Umsetzung bei Temperaturen von -70°C bis 150°C durchführt.

4. Verfahren nach Ansprüchen 2 und 3, dadurch gekennzeichnet, daß man die Nitroalkane und den Formaldehyd oder dessen Derivate im Verhältnis von 1:2-8 einsetzt.

5. Mikrobizides Mittel zum Schutz technischer Materialien, enthaltend Fluoralkyl enthaltende Dimethylolnitromethane der Formel

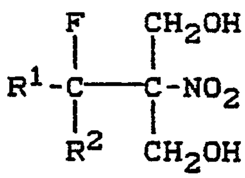 ,

worin $R^1$ und $R^2$ gleich oder verschieden sind und für Wasserstoff, Halogen, Alkyl oder Halogenalkyl stehen.

6. Mikrobizides Mittel enthaltend die Dimethylnitromethane in einer Menge von 1 bis 95 %.

7. Verwendung von fluoralkylhaltigen Dimethylolnitromethanen der Formel

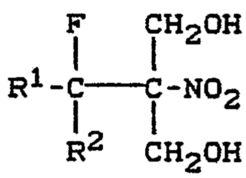 ,

worin $R^1$ und $R^2$ gleich oder verschieden sind und für Wasserstoff, Halogen, Alkyl oder Halogenalkyl stehen,
als mikrobizides Mittel zum Schutz technischer Materialien.

## Revendications

1. Diméthylolnitrométhanes contenant des groupes fluor-alkyle et répondant à la formule

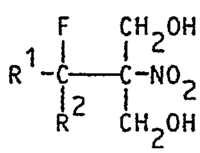

dans laquelle $R^1$ et $R^2$, ayant des significations identiques ou différentes, représentent chacun l'hydrogène, un halogène, un groupe alkyle ou halogénoalkyle, à l'exception du composé pour lequel $R^1$ et $R^2$ représentent le fluor.

2. Procédé de préparation des diméthylolnitrométhanes contenant des groupes fluoralkyle et répondant à la formule

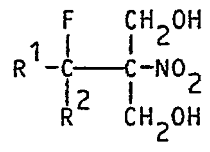

dans laquelle $R^1$ et $R^2$, ayant des significations identiques ou différentes, représentent chacun l'hydrogène, un halogène, un groupe alkyle ou halogénoalkyle, à l'exception du composé pour lequel $R^1$ et $R^2$ représentent le fluor, caractérisé en ce que l'on fait réagir des nitroalcanes de formule

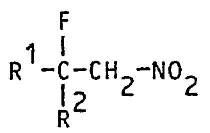

dans laquelle $R^1$ et $R^2$ ont les significations indiquées ci-dessus, avec le formaldéhyde de formule
$CH_2O$
ou des dérivés cycliques du formaldéhyde de formule

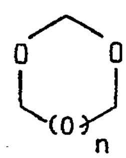

dans laquelle n et égal à 1 ou 2, ou avec des polyformaldéhydes de formule
$(CH_2{-}O)_n$,
dans laquelle n est un nombre supérieur à 10, en présence d'un solvant et/ou diluant et le cas échéant en présence d'une base.

3. Procédé selon la revendication 2, caractérisé en ce que l'on effectue la réaction à des températures de −70 à +150°C.

4. Procédé selon les revendications 2 et 3, caractérisé en ce que l'on met en œuvre les nitroalcanes et le formaldéhyde ou ses dérivés dans un rapport de 1:2 à 8.

5. Produit microbicide pour la protection des matières techniques, contenant des diméthylolnitrométhanes à groupes fluoralkyle qui répondent à la formule

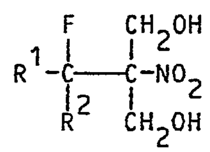

dans laquelle $R^1$ et $R^2$, ayant des significations identiques ou différentes, représentent chacun l'hydrogène, un halogène, un groupe alkyle ou halogénoalkyle.

6. Produit microbicide contenant les diméthylolnitrométhanes en quantité de 1 à 95%.

7. Utilisation des diméthylolnitromtéhanes contenant des groupes fluoralkyle et répondant à la formule

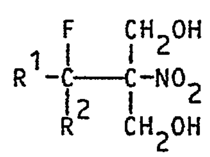

dans laquelle $R^1$ et $R^2$, ayant des significations identiques ou différentes, représentent chacun l'hydrogène, un halogène, un groupe alkyle ou halogénoalkyle, en tant que produits microbicides pour la protection des matières techniques.

## Claims

1. Fluoroalkyl-containing dimethylolnitromethanes of the formula

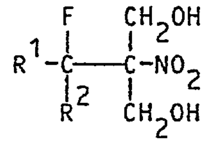

where $R^1$ and $R^2$ are identical or different and denote hydrogen, halogen, alkyl or halogen, alkyl or halogenalkyl, with the exception of the compound in which $R^1$ and $R^2$ represent fluorine.

2. Process for the preparation of fluoroalkyl-containing dimethylolnitromethanes of the formula

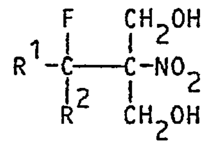

where $R^1$ and $R^2$ are identical or different and denote hydrogen, halogen, alkyl or halogenoalkyl, with the exception of the compound in which $R^1$ and $R^2$ represent fluorine, characterized in that nitroalkanes of the formula

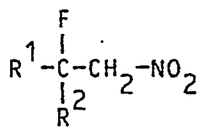

where $R^1$ and $R^2$ have the abovementioned meaning are reacted with formaldehyde, of the formula
$CH_2O$
or with cyclic formaldehyde derivatives of the formula

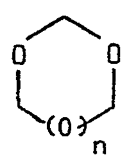

where n represents 1 or 2, or with polyformaldehyde of the formula
$(CH_2{-}O{-})_n$
where n represents a number > 10, in the presence of a solvent and/or diluent and if appropriate in the presence of a base.

3. Process according to Claim 2, characterized in that the reaction is carried out at temperatures from –70°C to 150°C.

4. Process according to Claims 2 and 3, characterized in that the nitroalkanes and the formaldehyde or its derivatives are employed in a ratio of 1:2–8.

5. Microbicidal agent for the protection of industrial materials, which contains fluoroalkyl-containing dimethylolnitromethanes of the formula

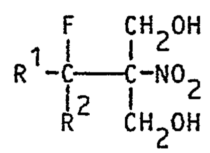

where $R^1$ and $R^2$ are identical or different and represent hydrogen, halogen, alkyl or halogenoalkyl.

6. Microbicidal agent which contains the dimethylnitromethanes in an amount of 1 to 95%.

7. Use of fluoroalkyl-containing dimethylolnitromethanes of the formula

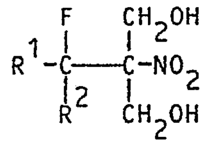

where $R^1$ and $R^2$ are identical or different and represent hydrogen, halogen, alkyl or halogenoalkyl, as a microbicidal agent for the protection of industrial materials.